# EUROPEAN PATENT SPECIFICATION

(11) **EP 3 342 440 B1**
(45) Date of publication and mention of the grant of the patent: **10.07.2019**
(21) Application number: 16207453.8
(22) Date of filing: 30.12.2016
(51) Int. Cl.: A61M 5/34, B29C 43/36

(54) **MEDICAL DEVICE, ASSEMBLY INCLUDING SAID MEDICAL DEVICE AND PROCESS FOR MANUFACTURING SUCH A MEDICAL DEVICE**
MEDIZINISCHE VORRICHTUNG, ANORDNUNG MIT BESAGTER MEDIZINISCHER VORRICHTUNG UND VERFAHREN ZUR HERSTELLUNG SOLCH EINER MEDIZINISCHEN VORRICHTUNG
DISPOSITIF MÉDICAL, ENSEMBLE COMPRENANT LEDIT ENSEMBLE ET PROCÉDÉ DE FABRICATION D'UN TEL DISPOSITIF MÉDICAL

(43) Date of publication of application: 04.07.2018
(73) Proprietor: Becton Dickinson France, 38800 Le Pont-de-Claix (FR)
(72) Inventor: BRUNEL, Julien, 38450 Vif (FR); CATENA, Mario, 38560 Jarrie (FR); AULETTO, Jean-Maurice, 38450 Vif (FR)
(74) Representative: Regimbeau

(56) References cited:
- WO-A2-2006/127843
- DE-A1-102013 015 820
- US-A- 6 033 385
- US-A1- 2012 245 564
- US-A1- 2016 003 391

## Description

### FIELD OF THE INVENTION

The present invention is defined by claim 1 and relates to a medical device for use in delivering a pharmaceutical composition, vitamins, a vaccine or any other type of medical solution to the body of a patient, and also relates to a process for manufacturing such a device.

In this application, the distal end of a component or apparatus must be understood as meaning the end furthest from the hand of the user and the proximal end must be understood as meaning the end closest to the hand of the user. As such, in this application, the distal direction must be understood as the direction of injection, and the proximal direction is the opposite direction, i.e. the direction towards the hand of the user.

### BACKGROUND OF THE INVENTION

Drug delivery devices such as syringes typically include a container and/or a reservoir for containing a medical solution having an end piece in a form of a longitudinal tip defining a fluid path through which the medical solution is expelled from the container and/or reservoir. Drug delivery devices are preferably made of glass that is a material known for its high chemical passivity, its low gas permeability and high transparency, which allows an extended storage and an easy inspection. However, this longitudinal tip does not allow parenteral administration by itself and must either comprise a staked needle or an adaptor allowing the connection of the drug delivery device to a connector such as a needle hub or an intravenous (IV) line or a needle hub directly connected to the longitudinal tip. During use of such devices, it is important that the adaptor and/or the needle hub is securely connected to the longitudinal tip of the device for the medical staff and patients' safety but also in order to deliver the entire dose of the medical solution contained in the container, to avoid leakage of liquid transferred through the devices. In the case that the longitudinal tip of the syringe is closed by a cap, it is important that the cap is securely closed onto the tip, avoiding leakage or contamination of the drug.

WO 2016/050699 discloses a drug delivery device provided with a gripping surface on a specific portion of an outer surface of the longitudinal tip (the end piece) in order to provide a reliable connection between said longitudinal tip and the adaptor. WO 2016/050699 also teaches that the gripping surface can be formed by applying a ceramic coating onto the longitudinal tip or alternatively through modification of the outer surface of the longitudinal tip by using abrasion technique, plasma or laser treatment.

However, the ceramic coating is porous and thus reduces tightness of the connection between the adaptor and the longitudinal tip. The above-mentioned alternative approach has also the drawback of generating particles and poses a risk of contamination of the medical solution. In addition, the disclosed technique makes it rather difficult to reproduce a specific shape of the gripping surface, and therefore, tightness of connection between the adaptor and the longitudinal tip may vary among the individual devices. In addition, this technique of adding a ceramic coating on the tip creates stress and constraints on the tip and may weaken the tip of the syringe, causing breakage but also, as the coating added on the tip makes the tip bigger, it is no longer conform to ISO standards.

A further prior art medical device is known from DE 10 2013 015 820 A1 disclosing a syringe with a Luer lock connector for attaching a tube to a terminal discharge channel opening for dispensing the content of the syringe.

In view of the above, an object of the invention is to improve reliability of the connection between the longitudinal tip of the medical device and an element to be attached to said longitudinal tip, such as an adaptor and/or directly a needle hub and/or a cap.

### SUMMARY OF THE INVENTION

According to a first aspect of the present invention, there is provided a device for use in delivering medical solution to a body of a patient, comprising: an elongated main body defining a reservoir for containing a medical solution; an end piece located at the distal end of the reservoir and extending axially therefrom, the end piece in a form of a longitudinal tip defining a fluid path extending axially through the longitudinal tip and in fluid communication with the reservoir; and a gripping portion formed on an outer surface of the longitudinal tip, the gripping portion includes from three to eight ribs integrally formed with the longitudinal tip and extending around a longitudinal axis of said longitudinal tip, characterized in that the main body and longitudinal tip are made of glass, and the gripping portion has an average roughness of 0.5 µm to 3 µm.

According to a second aspect of the present invention, in the device according to the first aspect, the ribs are arranged at predetermined intervals on the outer surface of the longitudinal tip.

According to a third aspect of the present invention, in the device according to the first or second aspect, each of the ribs has a rounded profile.

According to a fourth aspect of the invention, in the device according to any one of the first to third aspects, the distance between adjacent ribs is comprised between 0.4 and 0.8 mm.

According to a fifth aspect of the present invention, in the device according to any one of the first to fourth aspects, each of the ribs has a height of 0.01 mm to 0.1 mm.

According to a sixth aspect of the present invention, in the device according to any one of the first to fifth aspects, the ribs are axially spaced apart from each other over a total length of 3mm to 6mm.

According to a seventh aspect of the present invention, in the device according to any one of the first to sixth aspects, the longitudinal tip has a frustoconical shape having a smaller diameter at its distal end than at its proximal end.

According to an eighth aspect of the present invention, there is provided an assembly for use in delivering medical solution into a body of a patient, the assembly comprising: the device according to any one of the first to seventh aspects; and an additional element fitted onto the gripping portion of the longitudinal tip.

According to ninth aspect of the present invention, in the assembly according to the eighth aspect, the additional element is a needle hub.

According to a tenth aspect of the present invention, in the assembly according to the eighth aspect, the additional element is a tip cap.

According to an eleventh aspect of the present invention, there is provided a process for manufacturing the device as described above, the process comprising:
at a final forming tip station where the final shape of a longitudinal tip is to be obtained, inserting a longitudinal tip of the device between a pair of forming wheels rotating in opposite directions with regard to the longitudinal tip, the material of the longitudinal tip being at a temperature above its softening point; and
pressing the wheels against an outer surface of the longitudinal tip,
wherein the wheels are provided with recesses on their respective outer surfaces that come in contact with the longitudinal tip when the longitudinal tip is formed, each rib being formed by the softened material of the tip filling a respective recess of the wheels.

Thus, the ceramic coating on the longitudinal tip of the existing medical device is replaced with a gripping portion formed with three to eight ribs, extending around a longitudinal axis of the longitudinal tip which is made of glass and the gripping portion has an average roughness of 0.5 µm to 3 µm.

This gripping portion can ensure tight connection between the longitudinal tip and an additional element to be attached to the tip and at the same time requires relatively small force to pull the additional element apart from the longitudinal tip when the device is in use.

In the case where the syringe tip is shaped by pressing rotating forming wheels against the outer surface of the tip, the specific shape of the tip can be easily replicated in a precise manner.

### BRIEF DESCRIPTION OF THE FIGURES

The present invention will be described in further details in the following description with reference to the accompanying figures, in which:
Figure 1 shows a medical device such as a syringe according to an embodiment of the invention;
Figure 2 is a longitudinal section view showing the medical device;
Figure 3 is a partial enlarged view showing a tip portion of the medical device;
Figures 4A and 4B show an assembly including the medical device and a needle hub attached to the longitudinal tip of the medical device;
Figures 5A and 5B show an assembly including the medical device and a tip cap attached to the longitudinal tip of the medical device;
Figure 6 is an enlarged view showing a pair of forming wheels; and
Figure 7 schematically shows the step of forming the longitudinal tip according to an embodiment; and
Figures 8A-8C show comparative experimental results of the leak rate at the interface between the tip and a needle hub, the leak force at the interface between the tip and a tip cap and the tip cap pull out force for a gripping portion according to the invention and the gripping portion of the prior art showing a ceramic coating.

### DETAILED DESCRIPTION OF EMBODIMENTS OF THE INVENTION

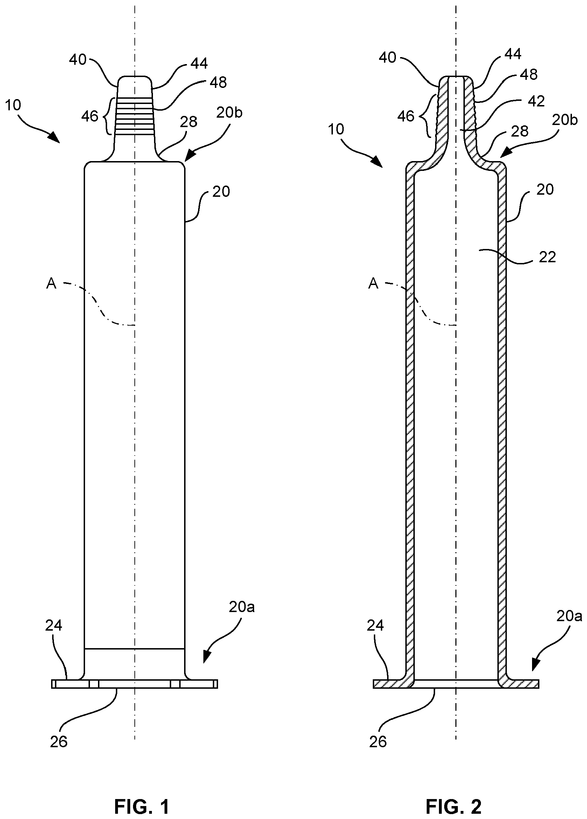

Figure 1 shows a medical device 10 according to an embodiment of the invention. Figure 2 is a longitudinal section view of the medical device 10. As shown in Figures 1 and 2, the medical device 10 is a generally elongated hollow element extending along an axis A. The medical device 10 includes an elongated main body 20 and a longitudinal tip 40 extending distally from the main body 20.

The main body 20 extends axially (i.e., along the axis A) and has a generally tubular shape. The main body 20 defines a reservoir 22 for containing a medical solution. The medical solution may include a pharmaceutical composition, vitamins, a vaccine, or any other type of injectable drugs.

The main body 20 has at the proximal end 20a a flange 24 extending radially outwardly from the main body 20. The main body 20 defines at the proximal end 20a an opening 26 which is generally concentric with the main body 20, so that the medical device 10 allows a plunger (not shown) with the help of a plunger rod (not shown) to advance within the reservoir 22 in a distal direction through the opening 26 in order to expel the medical solution contained in the reservoir 22.

The longitudinal tip 40 also has a generally tubular shape with a smaller diameter than the main body 20. The longitudinal tip 40 is smoothly connected to the distal end 20b of the main body 20 by a shoulder portion 28. The longitudinal tip 40 defines a fluid path 42 extending axially through the longitudinal tip 40. The fluid path 42 is in fluid communication with the reservoir 22. Accordingly, the medical solution contained in the reservoir 22 is expelled through the fluid path 42 of the longitudinal tip 40. The main body 20 and the longitudinal tip 40 are made of glass and integrally formed with each other.

The medical device 10 is used to deliver the medical solution contained in the reservoir 22 to the body of a patient. The medical device 10 may be used with an additional element connected to the distal end thereof, in particular to the longitudinal tip 40. In an embodiment of the invention, the longitudinal tip 40 may be designed such that an adaptor can be fitted thereto to receive one end of an intravenous line or a needle hub. In another embodiment of the invention, a needle hub may be directly attached to the longitudinal tip 40 of the medical device 10. In yet another embodiment, other accessories such as a tip cap may be attached to the longitudinal tip 40.

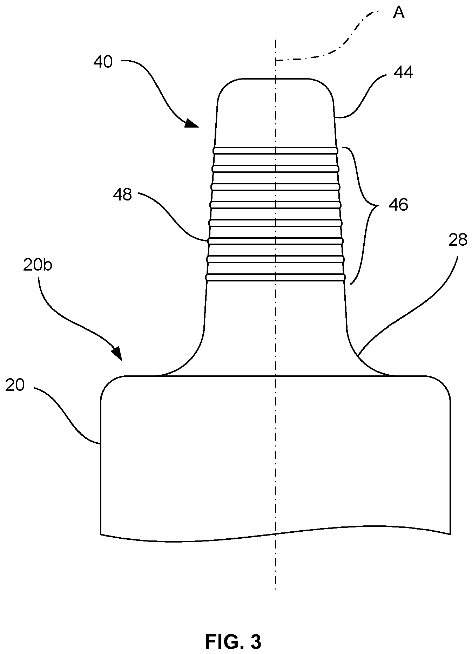

With additional reference to Figure 3, the longitudinal tip 40 has a gripping portion 46 on its outer surface 44. Preferably, the gripping portion 46 may extend over a portion closest to the distal end of the longitudinal tip 40.

The gripping portion 46 includes a plurality of annular ribs 48 (e.g., eight ribs in the illustrated embodiment). The ribs 48 extend around the axis A of the longitudinal tip 40. The ribs 48 have generally the same shape as each other, although the outer diameters of the ribs 48 slightly differ from each other, since the longitudinal tip 40 has a frustoconical shape with a smaller diameter at the distal end than at the proximal end.

According to an embodiment, an average roughness Ra (arithmetical mean roughness) of the gripping portion 46, measured with a roughometer, ranges between 0.5 µm to 3 µm. In contrast, an average roughness of the bare glass (i.e. without the gripping portion 46) is about 0.005 µm.

The gripping portion 46 may include from three to eight ribs 48 in order to achieve the tight connection between the longitudinal tip 40 and an additional element to be securely fitted onto the longitudinal tip 40, while at the same time requiring relatively small force to pull the additional element apart from the longitudinal tip 40 when needed. The length of the gripping portion may extend from 3 to 6 mm. In an embodiment, the longitudinal tip 40 is provided with eight ribs 48 spaced apart from each other over a total length of 5 mm. The distance between adjacent ribs may be comprised between 0.4 and 0.8 mm, for example about 0.5 mm. Said distance is considered as the distance between the tops of adjacent ribs.

The height of the ribs 48 is between 0.01 mm and 0.1 mm, preferably between 0.03 and 0.07 mm, for example around 0.05 mm.

If the longitudinal tip 40 is formed with too many ribs 48, the pull-out force which is the force required to pull an additional element apart from the longitudinal tip 40 tends to be too high, which makes it difficult to prepare the medical device 10 for use. On the other hand, if the number of ribs 48 is insufficient, the gripping force between the additional element and the longitudinal tip 40 is too weak, reducing reliability of the connection between the additional element and the medical device 10.

Moreover, the medical device 10 according to the present embodiment does not require an additional material such as a ceramic coating or any other type of coating, and therefore, there is no risk of generating particles which would come off from the longitudinal tip 40 coated with a ceramic coating, thus potentially resulting in contamination of the medical solution stored in the reservoir 22. Furthermore, as compared to the existing technique in which a ceramic coating is applied on a longitudinal tip, the longitudinal tip 40 is not weakened in the gripping portion while achieving sufficient gripping force between the longitudinal tip 40 and an additional element to be fitted onto the longitudinal tip 40.

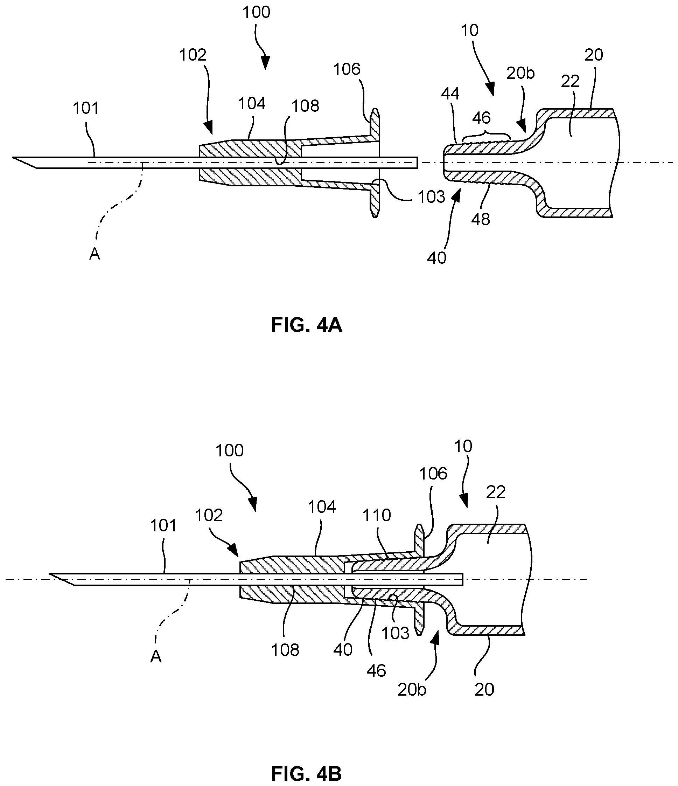

Figures 4A and 4B show an assembly 100 including the medical device 10 and a needle hub 102 attached directly to the longitudinal tip 40 of the medical device 10. The needle hub 102 includes a hub 104 and may or may not show a flange 106. The hub 104 includes a lumen 108 through which the hub 104 holds a needle 101. The needle hub 102 may be made of a plastic or aluminum material or any other material suitable for such device. The flange 106 aims to facilitate the action of pulling the needle hub 102 apart from the medical device 10.

The hub 104 is also formed with a cavity 103 sized to be fitted onto the longitudinal tip 40 of the medical device 10. Specifically, in an assembled state, the outer surface 44 of the longitudinal tip 40 is in tight contact with a circumferential inner wall of the cavity 103 on its gripping portion 46, thereby establishing tight connection between the needle hub 102 and the longitudinal tip 40.

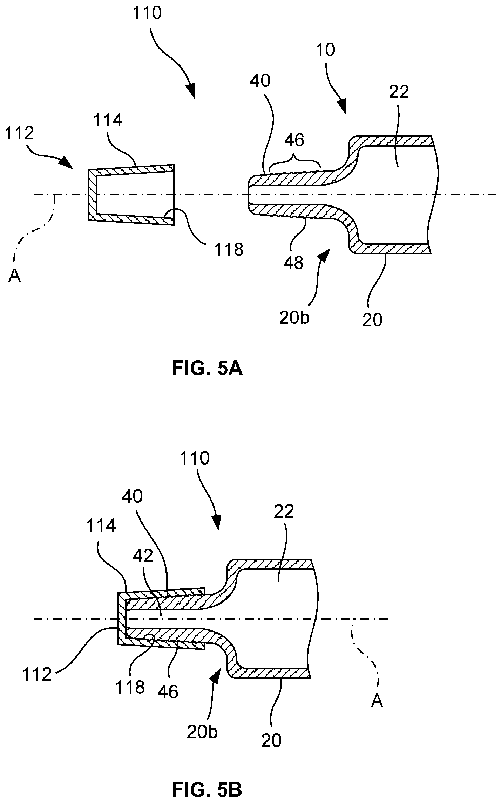

Figures 5A and 5B show an assembly 110 including the medical device 10 and a tip cap 112 attached to the longitudinal tip 40 of the medical device 10. The tip cap 112 is intended to protect the content of the reservoir 22 from contamination. The tip cap 112 includes a body part 114 in the form of a cup having a closed distal end. The body part 114 defines a cavity 118 which is open at the proximal end. The cavity 118 is sized to be engaged with the gripping portion 46 of the longitudinal tip 40. The tip cap 112 is designed to close the distal end of the fluid path 42 of the longitudinal tip 40 when fully assembled to the medical device10 as shown in Figure 5B. The tip cap 112 may be made of a material suitable for closing the fluid path 42.

Suitable materials for the tip cap of the invention include natural rubber, acrylate-butadiene rubber, cis-polybutadiene, chloro or bromobutyl rubber, chlorinated olyethylene elastomers, polyalkylene oxide polymers, ethylene vinyl acetate, fluorosilicone rubbers, hexafluoropropylene-vinylidene fluoridetetrafluoroethyleneterpolymers, butyl rubbers, polyisobutene, synthetic polyisoprene rubber, silicone rubbers, styrene-butadiene rubbers, tetrafluoroethylene propylene copolymers, thermoplastic-copolyesters, thermo-plastic elastomers, or the like or a combination thereof.

According to the present embodiment, thanks to the gripping portion 46 of the longitudinal tip 40 provided with a plurality of ribs 48, the force required to pull the needle hub 102 or the tip cap 112 apart from the medical device 10 is relatively small, but at the same time, a reliable connection between the medical device 10 and the needle hub 102 or the tip cap 112 is established.

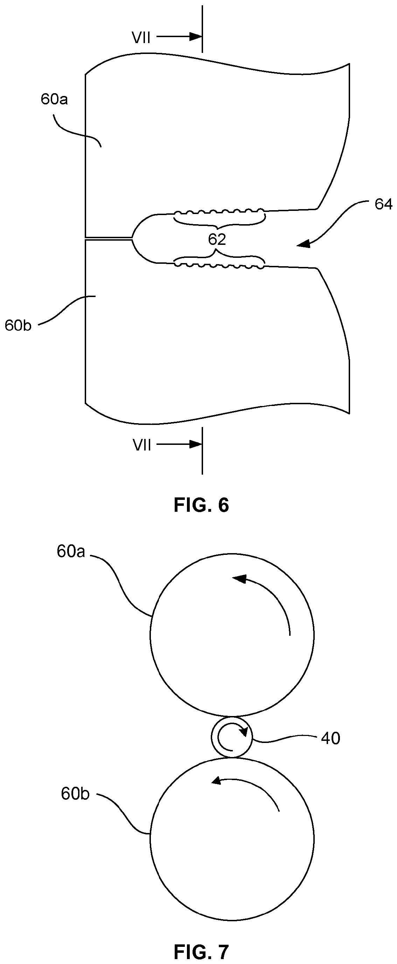

In addition, a method of forming the gripping portion 46 on the longitudinal tip 40 of the medical device 10 will be explained below with reference to Figures 6 and 7. According to an embodiment, the longitudinal tip 40 may be shaped by using a pair of rotating forming wheels.

The medical device 10 may be shaped by forming a glass tube. The method of forming the main body 20 and an end tip extending distally from the main body 20 is well known in the art of syringes. On the other hand, according to the present embodiment, the longitudinal tip 40 is shaped by applying pressure from the two rotating wheels onto the outer surface 44 of the longitudinal tip 40.

Figure 6 shows a pair of rotating forming wheels 60a and 60b used to form an outer shape of the longitudinal tip 40 including the gripping portion 46. Each of the wheels 60a and 60b has a plurality of recesses 62 on its outer surface which faces the other wheel 60a or 60b. The recesses 62 are sized and arranged onto the outer surface of the wheels correspondingly to the ribs 48 to be formed on the longitudinal tip 40.

In an embodiment, the recesses 62 may have a rounded profile without sharp edges. This facilitates the softened glass material of the longitudinal tip 40 to fill the entire volume of the recesses 62, thereby precisely replicating the shape of the longitudinal tip 40 including the ribs 48. It should be noted that, according to this embodiment, as a consequence, the ribs 48 also have a rounded profile correspondingly to the recesses 62.

The wheels 60a and 60b are installed on purpose at the final forming tip station where the final shape of the longitudinal tip 40 of the medical device 10 is formed. During the forming of the longitudinal tip 40 of the medical device 10 that is introduced in a space 64 between the two wheels 60a and 60b, as visible on Figure 6, the longitudinal tip 40 is heated up to its softening point, e.g., to a temperature between 900°C and 1100°C and is therefore in the ideal state to be formed with the ribs 48.

As shown in Figure 7, the wheels 60a and 60b are both caused to rotate in opposite directions with respect to the longitudinal tip 40 which is itself rotating. In other words and as further explained below, the longitudinal tip 40 is rotating in one direction while both wheels 60a and 60b are rotating in the opposite direction. Figure 7 shows an exemplary arrangement of the rotating forming wheels 60a and 60b and the longitudinal tip 40 of the medical device 10. When the medical device 10 is rotating in a clockwise direction, it will cause both rotating forming wheels (60a, 60b) to rotate in the opposite direction, that is, in a counter-clockwise direction. If the medical device 10 is rotating in a counter-clockwise direction, it results in both rotating forming wheels to rotate in the clockwise direction. The wheels 60a and 60b and the longitudinal tip 40 are rotated by electric motors, respectively, in a known manner. In conventional processes, forming wheels are also used to form the longitudinal tip, but they have smooth surfaces (i.e. without any recess or projection).

Since the gripping portion 46 of the longitudinal tip 40 is formed in the same process step performed at the final tip station for shaping the longitudinal tip 40, there is no need for an extra step to manufacture the longitudinal tip 40 of the medical device 10 of the present invention. Besides, the longitudinal tip 40 including the ribs 48 is formed by simply deforming the glass, thereby preventing small particles from being generated during the manufacturing process.

Furthermore, the ribs 48 on the longitudinal tip 40 can be easily modified in size (width and height), shape and location since they correspond to recesses 62 performed on the wheels 60a and 60b which are subject to modification as necessary.

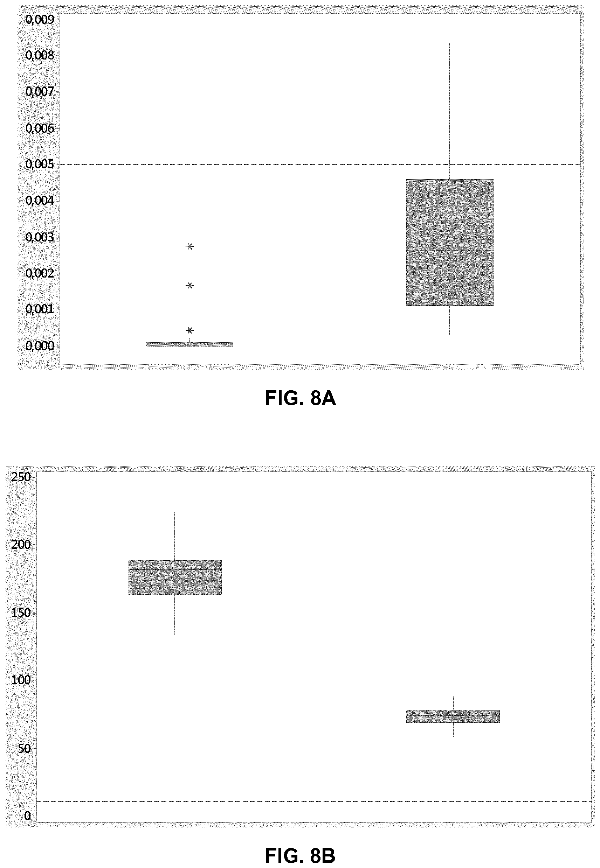

Figure 8A shows comparative experimental results of the leak rate (Pa.m³/s) at the interface between the tip and a needle hub.

The pressure decay test method uses ATEQ™ (ATEQ F5200 or F620) leak tester.

The test method, which is based on ISO 80 369-7, consists in applying a pressure of 3 bars during 15 seconds into the syringe whose tip is coupled to the needle hub. The maximum acceptable leak rate is of 0,005000 Pa.m³/s.

Two types of gripping portions of glass syringes have been tested, each on 30 samples:
- a gripping portion with eight ribs (height: 0.05 mm; distance between adjacent ribs: 0.55 mm) according to the invention;
- a gripping portion with a ceramic coating (with a thickness between 1 µm and 30 µm) (prior art);
   FIG. 8A shows the results obtained with the above-described method (left: gripping portion according to the invention; right: gripping portion with a ceramic coating). With this method, the samples with the gripping portion according to the invention are way below the maximum acceptable rate and thus comply with ISO 80 369-7, with a much smaller leak rate than with the other gripping portion.

These comparative results thus show that the gripping portion according to the invention is much more efficient than the gripping portion showing the ceramic coating for preventing leak at the needle hub / tip interface.

FIG. 8B shows comparative results of the leak force (N) at the interface between the tip and a tip cap, for a tip with a gripping portion according to the invention (same configuration as for FIG. 8A, left), and with a gripping portion formed of a ceramic layer (prior art, right).

The test method consists in filing a syringe whose tip is closed by a tip cap with WFI (Water For Injection), then insert a plunger stopper into the syringe and further assemble with a plunger rod. Then, a traction/compression bench (LLOYD LRX PLUS) is used and a pressure is applied onto the plunger rod till leak. The leak force (N) is then recorded.

The leak force is greater with the gripping portion according to the invention.

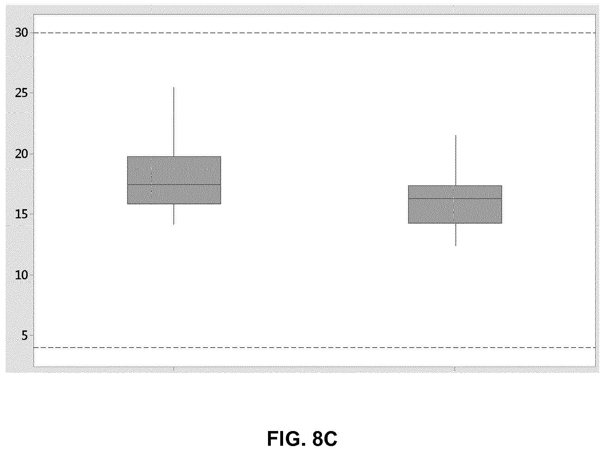

FIG. 8C shows comparative results of the tip cap pull out force (N) for a tip with a gripping portion according to the invention (same configuration as for FIG 8A, left), and with a gripping portion showing a ceramic layer (prior art, right).

The test method consists in closing a syringe tip with a tip cap. Then, using a traction/compression bench (LLOYD LRX PLUS), the tip cap is disassembled from the glass tip. Force versus displacement is then recorded.

The pull out force is slightly greater with the gripping portion according to the invention, which shows that the connection between the tip and the tip cap is improved.

### REFERENCES

WO 2016/050699, DE 10 2013 015 820 A1

## Claims

1. Medical device (10) for use in delivering a medical solution into the body of a patient, comprising:
an elongated main body (20) defining a reservoir (22) for containing a medical solution;
a longitudinal tip (40) extending axially and distally from the main body (20), the longitudinal tip (40) defining a fluid path (42) extending axially through the longitudinal tip (40) and in fluid communication with the reservoir (22); and
a gripping portion (46) formed on an outer surface (44) of the longitudinal tip (40),
the gripping portion (46) includes from three to eight ribs (48) integrally formed with the longitudinal tip (40), each rib (48) extending around a longitudinal axis (A) of the longitudinal tip (40),
**characterized in that** the main body (20) and longitudinal tip (40) are made of glass, and the gripping portion (46) has an average roughness of 0.5 µm to 3 µm.

2. Device (10) according to claim 1, wherein the ribs (48) are arranged at predetermined intervals on the outer surface (44) of the longitudinal tip (40).

3. Device (10) according to claim 1 or 2, wherein each of the ribs (48) has a rounded profile.

4. Device (10) according to any one of claims 1 to 3, wherein the distance between adjacent ribs (48) is comprised between 0.4 and 0.8 mm.

5. Device (10) according to any one of claims 1 to 4, wherein each of the ribs (48) has a height of 0.01 mm to 0.1 mm.

6. Device (10) according to any one of claims 1 to 5, wherein the ribs (48) are axially spaced apart from each other over a total length of 3 to 6 mm.

7. Device (10) according to any one of claims 1 to 6, wherein the longitudinal tip (40) has a frustoconical shape having a smaller diameter at a distal end than a proximal end.

8. Assembly (100, 110) for use in delivering medical solution to a body of a patient, the assembly (100,110) comprising:
the device (10) according to any one of claims 1 to 7; and
an additional element (102, 112) fitted onto the gripping portion (46) of the longitudinal tip (40).

9. Assembly (100) according to claim 8, wherein the additional element is a needle hub (102).

10. Assembly (110) according to claim 8, wherein the additional element is a tip cap (112).

11. Process for manufacturing the device (10) according to any one of claims 1 to 7, the process comprising:
at a final forming tip station where the final shape of a longitudinal tip (40) is to be obtained, inserting a longitudinal tip (40) of the device (10) between a pair of forming wheels (60a, 60b) rotating in opposite directions with regard to the longitudinal tip, the material of the longitudinal tip being at a temperature above its softening point; and
pressing the wheels (60a, 60b) against an outer surface (44) of the longitudinal tip (40),
wherein the wheels (60a, 60b) are provided with recesses (62) on their respective outer surfaces that come in contact with the longitudinal tip (40) when the longitudinal tip (40) is formed, each rib (48) being formed by the softened material of the tip filling a respective recess (62) of the wheels.

## Patentansprüche

1. Medizinische Vorrichtung (10) zur Verwendung bei der Abgabe einer medizinischen Lösung in den Körper eines Patienten, umfassend:
einen länglichen Hauptkörper (20), der einen Behälter (22) zum Aufnehmen einer medizinischen Lösung definiert;
eine Längsspitze (40), die sich axial und distal vom Hauptkörper (20) erstreckt, wobei die Längsspitze (40) einen Fluidpfad (42) definiert, der axial durch die Längsspitze (40) verläuft und in Fluidverbindung mit dem Behälter (22) steht; und
einen Greifabschnitt (46), der an einer Außenfläche (44) der Längsspitze (40) ausgebildet ist, wobei der Greifabschnitt (46) drei bis acht Rippen (48) aufweist, die integral mit der Längsspitze (40) ausgebildet sind, wobei sich jede Rippe (48) um eine Längsachse (A) der Längsspitze (40) erstreckt,
**dadurch gekennzeichnet, dass** der Hauptkörper (20) und die Längsspitze (40) aus Glas hergestellt sind, und der Greifabschnitt (46) eine durchschnittliche Rauheit von 0,5 µm bis 3 µm aufweist.

2. Vorrichtung (10) nach Anspruch 1, wobei die Rippen (48) in im Voraus bestimmten Abständen auf der Außenfläche (44) der Längsspitze (40) angeordnet sind.

3. Vorrichtung (10) nach Anspruch 1 oder 2, wobei jede der Rippen (48) ein abgerundetes Profil aufweist.

4. Vorrichtung (10) nach einem der Ansprüche 1 bis 3, wobei der Abstand zwischen benachbarten Rippen (48) zwischen 0,4 und 0,8 mm beträgt.

5. Vorrichtung (10) nach einem der Ansprüche 1 bis 4, wobei jede der Rippen (48) eine Höhe von 0,01 mm bis 0,1 mm aufweist.

6. Vorrichtung (10) nach einem der Ansprüche 1 bis 5, wobei die Rippen (48) über eine Gesamtlänge von 3 bis 6 mm axial voneinander beabstandet sind.

7. Vorrichtung (10) nach einem der Ansprüche 1 bis 6, wobei die Längsspitze (40) eine kegelstumpfförmige Form mit einem kleineren Durchmesser an einem distalen Ende als an einem proximalen Ende aufweist.

8. Anordnung (100, 110) zur Verwendung bei der Abgabe einer medizinischen Lösung an einen Körper eines Patienten, wobei die Anordnung (100, 110) umfasst:
die Vorrichtung (10) nach einem der Ansprüche 1 bis 7; und
ein zusätzliches Element (102, 112), das am Greifabschnitt (46) der Längsspitze (40) angebracht ist.

9. Anordnung (100) nach Anspruch 8, wobei das zusätzliche Element eine Nadelnabe (102) ist.

10. Anordnung (110) nach Anspruch 8, wobei das zusätzliche Element eine Spitzenkappe (112) ist.

11. Verfahren zur Herstellung der Vorrichtung (10) nach einem der Ansprüche 1 bis 7, wobei das Verfahren umfasst:
an einer Spitzen-Endformungsstation, an der die Endform einer Längsspitze (40) erlangt werden soll, Einfügen einer Längsspitze (40) der Vorrichtung (10) zwischen einem Paar von Formungsrädern (60a, 60b), die sich in Bezug auf die Längsspitze in entgegengesetzten Richtungen drehen, wobei das Material der Längsspitze eine Temperatur oberhalb ihres Erweichungspunktes aufweist; und
Drücken der Räder (60a, 60b) gegen eine Außenfläche (44) der Längsspitze (40),
wobei die Räder (60a, 60b) an ihren jeweiligen Außenflächen mit Ausnehmungen (62) versehen sind, die mit der Längsspitze (40) in Kontakt kommen, wenn die Längsspitze (40) ausgebildet wird, wobei jede Rippe (48) durch das erweichte Material der Spitze gebildet wird, das eine jeweilige Ausnehmung (62) der Räder ausfüllt.

## Revendications

1. Dispositif médical (10) destiné à être utilisé pour administrer une solution médicale dans le corps d'un patient, comprenant :
un corps principal allongé (20) définissant un réservoir (22) pour contenir une solution médicale ;
une extrémité longitudinale (40) s'étendant de manière axiale et distale à partir du corps principal (20), l'extrémité longitudinale (40) définissant une trajectoire de fluide (42) s'étendant de manière axiale à travers l'extrémité longitudinale (40) et en communication de fluide avec le réservoir (22) ; et
une partie de préhension (46) formée sur une surface externe (44) de l'extrémité longitudinale (40), la partie de préhension (46) comprend de trois à huit nervures (48) formées de manière solidaire avec l'extrémité longitudinale (40), chaque nervure (48) s'étendant autour d'un axe longitudinal (A) de l'extrémité longitudinale (40),
**caractérisé en ce que** le corps principal (20) et l'extrémité longitudinale (40) sont réalisés à partir de verre, et la partie de préhension (46) a une rugosité moyenne de 0,5 µm à 3 µm.

2. Dispositif (10) selon la revendication 1, dans lequel les nervures (48) sont agencées à des intervalles prédéterminés sur la surface externe (44) de l'extrémité longitudinale (40).

3. Dispositif (10) selon la revendication 1 ou 2, dans lequel chacune des nervures (48) a un profil arrondi.

4. Dispositif (10) selon l'une quelconque des revendications 1 à 3, dans lequel la distance entre les nervures (48) adjacentes est comprise entre 0,4 et 0,8 mm.

5. Dispositif (10) selon l'une quelconque des revendications 1 à 4, dans lequel chacune des nervures (48) a une hauteur de 0,01 mm à 0,1 mm.

6. Dispositif (10) selon l'une quelconque des revendications 1 à 5, dans lequel les nervures (48) sont axialement espacées les unes des autres sur une longueur totale de 3 à 6 mm.

7. Dispositif (10) selon l'une quelconque des revendications 1 à 6, dans lequel l'extrémité longitudinale (40) a une forme tronconique ayant un plus petit diamètre au niveau d'une extrémité distale qu'au niveau d'une extrémité proximale.

8. Ensemble (100, 110) destiné à être utilisé pour administrer une solution médicale dans le corps d'un patient, l'ensemble (100, 110) comprenant :
le dispositif (10) selon l'une quelconque des revendications 1 à 7 ; et
un élément supplémentaire (102, 112) monté sur la partie de préhension (46) de l'extrémité longitudinale (40).

9. Ensemble (100) selon la revendication 8, dans lequel l'élément supplémentaire est un raccord d'aiguille (102).

10. Ensemble (110) selon la revendication 8, dans lequel l'élément supplémentaire est un capuchon de pointe (112).

11. Procédé pour fabriquer le dispositif (10) selon l'une quelconque des revendications 1 à 7, le procédé comprenant les étapes suivantes :
dans un poste de formage final d'une extrémité où la forme définitive d'une extrémité longitudinale (40) doit être obtenue, insérer une extrémité longitudinale (40) du dispositif (10) entre une paire de roues de formage (60a, 60b) tournant dans des directions opposées par rapport à l'extrémité longitudinale, le matériau de l'extrémité longitudinale étant à une température supérieure à son point de ramollissement ; et
comprimer les roues (60a, 60b) contre une surface externe (44) de l'extrémité longitudinale (40),
dans lequel les roues (60a, 60b) sont prévues avec des évidements (62) sur leurs surfaces externes respectives qui viennent en contact avec l'extrémité longitudinale (40) lorsque l'extrémité longitudinale (40) est formée, chaque nervure (48) étant formée par le matériau ramolli de l'extrémité qui remplit un évidement (62) respectif des roues.
